# FASCICULE DE BREVET EUROPEEN

(11) **EP 2 150 191 B1**
(45) Date de publication et mention de la délivrance du brevet: **02.07.2014**
(21) Numéro de dépôt: 08805611.4
(22) Date de dépôt: 27.05.2008
(51) Int. Cl.: A61B 18/02

(54) **DISPOSITIF CRYOGENIQUE A USAGE CHIRURGICAL**
KRYOGENE VORRICHTUNG ZUR CHIRURGISCHEN VERWENDUNG
CRYOGENIC DEVICE FOR SURGICAL USE

(30) Priorité: 31.05.2007 FR 0755370
(43) Date de publication de la demande: 10.02.2010
(73) Titulaire: Phakos, 93100 Montreuil (FR)
(72) Inventeur: AUMAITRE, Olivier, F-93100 Montreuil (FR)
(74) Mandataire: Novagraaf Technologies
(86) Numéro de dépôt international: PCT/FR2008/000720
(87) Numéro de publication internationale: WO 2009/004156

(56) Documents cités:
- WO-A-00/32126
- WO-A-99/37226
- US-A- 3 696 813
- US-A- 5 324 286
- US-A1- 2006 004 350

## Description

L'invention concerne un dispositif cryogénique pour des applications médicales. L'invention concerne plus particulièrement un dispositif cryogénique à usage unique pour la chirurgie ophtalmologique.

Dans l'art antérieur, il est connu des dispositifs cryogéniques pour des applications médicales. Ces dispositifs sont notamment utilisés dans le domaine de la cryochirurgie ophtalmologique pour traiter notamment les décollements de la rétine en générant un point froid permettant de créer une cicatrice adhésive, par brûlure des tissus, entre la rétine et la choroïde.

Les dispositifs de l'art antérieur se composent généralement d'un conduit d'alimentation connectée à une source de gaz cryogénique (protoxyde d'azote, dioxyde de carbone...), d'un conduit d'évacuation dudit gaz, d'un embout métallique, destiné à être mise en contact avec la zone à refroidir et pourvu d'une chambre de détente du gaz, et d'une buse d'injection qui conduit le gaz du conduit d'alimentation vers la chambre de détente.

Les dispositifs de l'art antérieur sont coûteux, longs et complexes à fabriquer, notamment en raison du nombre de pièces qui les composent. Par conséquent, afin de limiter les coûts, les dispositifs sont habituellement destinés à être réutilisés de nombreuses fois. Ceci impose que ces dispositifs soient capables de supporter les conditions de stérilisation en milieu hospitalier, soit Suivant les normes internationales, la stérilisation est obtenue par séjour en autoclave porté à 125 ° C pendant 10 minutes. La norme française correspondante est encore plus contraignante, puisqu'elle impose un séjour à 134°C pendant 20 minutes. Ces contraintes imposent des choix technologiques qui renchérissent encore la complexité et le coût des dispositifs. De plus, les dispositifs stérilisés selon cette procédure subissent au fil des stérilisations des dégradations qui imposent de fréquentes et coûteuses interventions de maintenance.

Le document US 2006/004350 décrit un dispositif cryogénique selon le préambule de la revendication 1.

Afin de pallier ces inconvénients, l'invention a pour but de proposer un dispositif cryogénique à usage unique qui soit fiable et dont la fabrication soit simple et peu onéreuse.

L'invention a également pour but de proposer un dispositif cryogénique léger, ergonomique, stérile et sécurisé.

À cet effet, et selon un premier aspect, l'invention propose un dispositif cryogénique à usage chirurgical permettant de refroidir une zone du corps comprenant :
- un embout comprenant une extrémité métallique destinée à être mise en contact avec la zone à refroidir et une chambre de détente ;
- un conduit d'alimentation de la chambre en gaz cryogénique ; et
- un conduit d'évacuation du gaz cryogénique de la chambre ;
le conduit d'alimentation et le conduit d'évacuation étant formées dans un tube flexible commun extrudé dont une extrémité est inséré à l'intérieur de l'embout.

Ainsi, le nombre de pièces composant le dispositif est limité. En outre, les opérations de montages sont simplifiées car le tube contenant les deux conduits, d'alimentation et d'évacuation, est directement inséré à l'intérieur de l'embout.

Avantageusement, l'embout est solidarisé à une extrémité du tube flexible par des moyens de collage.

Ainsi, le nombre de pièces est limité par l'utilisation de colle et le montage est particulièrement simple. L'utilisation de colle n'est naturellement possible que si la sonde est stérilisée à basse température. En pratique la stérilisation à basse température, par exemple à l'oxyde d'éthylène à 45°, nécessite des extracteurs qui ne sont pas disponibles dans les hôpitaux, et est donc pratiquée principalement en milieu industriel. En l'espèce, c'est parce que le montage du dispositif se trouve grandement simplifié par l'utilisation d'un tube unique à deux conduits que l'on peut envisager une fixation par une étape de collage simple, et que l'on peut ainsi diminuer globalement le coût de revient au point qu'un usage unique se justifie économiquement.

Dans un mode de réalisation, l'extrémité libre du tube flexible, insérée à l'intérieur de l'embout, est filetée afin de créer une rugosité à l'interface de collage entre l'embout et le tube flexible.

Avantageusement, le conduit d'alimentation présente une section inférieure au conduit d'évacuation.

Dans un mode de réalisation, le conduit d'alimentation présente sensiblement une forme de croissant et l'extrémité du conduit d'alimentation est biseautée.

Avantageusement, la buse d'injection est insérée à l'intérieur du conduit d'alimentation.

De préférence, la buse d'injection possède une extrémité conique d'injection.

Dans un mode de réalisation de l'invention, le dispositif cryogénique comprend un écrou pourvu d'un filetage intérieur et un filetage extérieur, un manche pourvu d'un filetage coopérant avec le filetage extérieur de l'écrou, l'extrémité du tube flexible étant équipé d'un filetage coopérant avec le filetage intérieur de l'écrou.

Avantageusement, le dispositif cryogénique comprend un manchon d'isolation recouvrant une portion de l'embout.

En pratique, le conduit d'alimentation est relié à une source de gaz cryogénique sous pression. Le conduit d'évacuation est quant à lui relié à un silencieux d'échappement du gaz vers l'extérieur.

D'autres objets et avantages de l'invention apparaîtront au cours de la description qui suit, faite en référence aux dessins annexés, dans lesquels :
- la figure 1 est une vue schématique d'un dispositif cryogénique selon l'invention ;
- la figure 2 est une vue schématique en coupe de l'extrémité de la sonde destinée à être mise en contact avec la zone du corps à refroidir, du dispositif de la figure 1 ;
- la figure 3 est une vue schématique d'un connecteur selon un mode de réalisation de l'invention ;
- la figure 4 est une vue schématique en coupe d'une buse d'injection du gaz ;
- la figure 5 est une vue détaillée de la zone A de la figure 4 ;
- la figure 6 est une vue schématique du tube flexible extrudé selon l'invention ;
- la figure 7 est une vue détaillée en coupe longitudinale de la zone B de la figure 6 ;
- la figure 8 est une vue en coupe transversale du tube flexible extrudé ;
- la figure 9 représente l'embout métallique ;
- la figure 10 est une vue en coupe longitudinale du manche du dispositif selon l'invention ;
- la figure 11 est une vue en perspective de l'écrou ; et
- la figure 12 est une vue en coupe longitudinale de l'écrou de la figure 11.

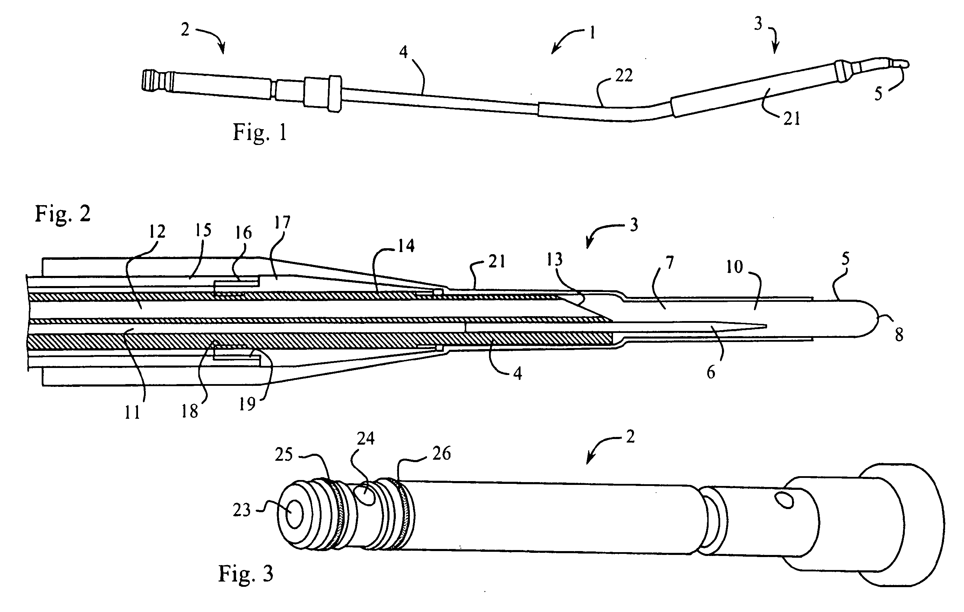

La figure 1 représente un dispositif cryogénique 1 selon l'invention comprenant un connecteur 2 destiné à être accouplé à un détendeur d'un réservoir de gaz liquéfié (protoxyde d'azote ou dioxyde de carbone) d'une part, une sonde ou cryode 3 d'autre part et un tube flexible 4 de transport du fluide cryogénique reliant le connecteur 2 à la cryode 3.

La cryode 3, représentée sur la figure 2, comprend un embout 5 creux, dont l'extrémité est destinée à être mise en contact avec la zone à refroidir, et une buse d'injection 6 débouchant dans une chambre 7 de l'embout 5 et projetant le gaz cryogénique contre l'extrémité 8 de la cryode 3. Ainsi, le gaz cryogénique refroidit les parois de l'embout 5 à l'extrémité 8 qui va être appliqué sur les tissus pour les congeler localement à une température inférieure à -50°C, et de préférence de l'ordre de -65°C à -90°C.

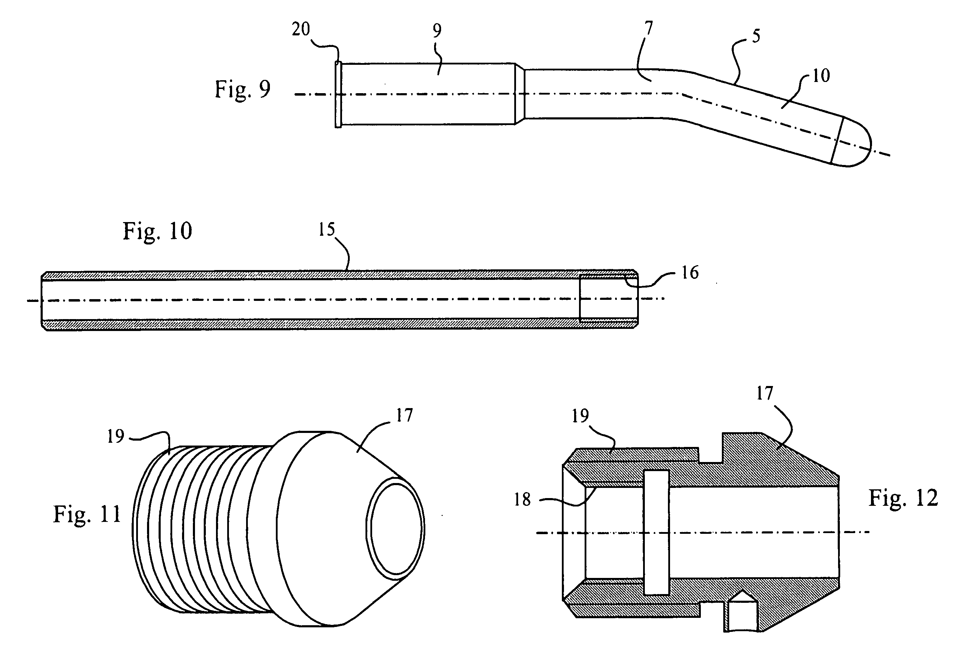

L'embout 5, représenté sur la figure 9. est réalisé dans un matériau métallique tel que l'acier inoxydable et ses parois forment une chambre 7 de détente du gaz constituée d'un chambrage 9 de large section prolongé par une canule 10. Avantageusement, afin d'augmenter l'ergonomie du dispositif, l'extrémité de l'embout 5 est légèrement courbée.

Par ailleurs, on notera que l'embout 5 est obtenu par emboutissages successifs. Ainsi, les parois de l'embout 5 présentent une épaisseur homogène afin de diminuer les risques d'explosion.

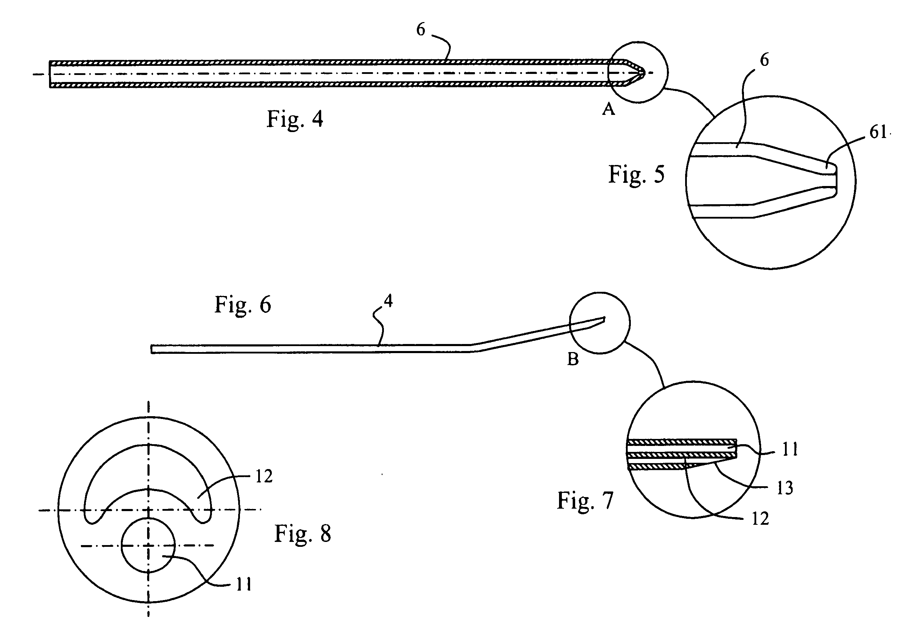

Le tube flexible 4, représenté sur la figure 6, est un tube extrudé pourvu d'un conduit d'alimentation 11 et d'un conduit d'évacuation 12 d'un gaz cryogénique.

L'extrémité du tube flexible 4 est insérée à l'intérieur de l'embout 5 et vient obturer l'extrémité de l'embout 5 afin de réaliser l'étanchéité de la cryode 3. L'embout 5 est solidarisé au tube flexible 4 par collage. Avantageusement, de manière à proposer une fixation fiable entre le tube 4 et l'embout, l'extrémité libre du tube 4 qui est insérée à l'intérieur de l'embout 5 est filetée afin de créer une rugosité à l'interface de collage entre l'embout 5 et le tube 4. La colle utilisée peut notamment être de la colle cyanolite.

Les deux conduits d'alimentation 11 et d'évacuation 12, représentés sur les figures 7 et 8, présentent des formes et des sections différentes.

Le conduit d'alimentation 11 est de forme sensiblement cylindrique et possède une section plus faible que celle du conduit d'évacuation 12 afin de maintenir le gaz sous pression avant son injection dans la chambre de détente 7.

Au contraire, de façon à maintenir la chambre de détente 7 en sous-pression, la section du conduit d'évacuation 12 est nécessairement plus importante afin de permettre l'évacuation du gaz qui s'est détendu dans ladite chambre 7. Avantageusement, afin d'optimiser la section du conduit d'évacuation 12 en fonction de la section du tube 4, le conduit d'évacuation 12 possède sensiblement une forme de croissant. En outre, la concavité du croissant est orientée vers le conduit d'alimentation 11.

En outre, afin d'augmenter la section du conduit d'évacuation 12 au niveau de son extrémité 13, la section du conduit d'évacuation est de préférence biseautée (voir figure 7).

Avantageusement, le tube 4 est réalisé dans une fibre synthétique polyamide, telle que le Rilsan ® présentant une excellente résistance à l'usure, aux chocs et aux vibrations.

Par ailleurs, le tube 4 présente, en outre, une gaine 14 extérieure de protection, réalisée en PVC. L'extrémité du tube 4 est dénudée de la gaine 14 qui ne pénètre pas, ainsi, à l'intérieur de l'embout 5.

Dans un mode de réalisation, la longueur du tube est comprise entre 1,5 à 2,5 mètres, et de préférence de l'ordre de 2 mètres afin de conférer une importante liberté de mouvement à l'opérateur par rapport à la source de gaz.

La buse 6, représentée de façon détaillée sur les figures 4 et 5, est insérée à l'intérieur du conduit d'alimentation 11. La buse 6 présente à son extrémité libre un cône d'injection 61, représenté sur la figure 5, permettant de vaporiser le gaz, à l'intérieur de la canule 10, contre l'extrémité 8 de l'embout 5. Dans un mode de réalisation de l'invention, la buse 6 est réalisée en acier inoxydable.

Afin de permettre la manipulation de la cryode, le dispositif est équipé d'un manche 15, illustré sur la figure 10, dont l'extrémité distale est pourvue d'un filetage 16 coopérant avec un écrou 17. De préférence, le manche 15 est réalisé dans un matériau isolant tel que l'aluminium.

L'écrou 17 est monté sur l'embout 5 et vient en butée contre une collerette 20 formée par la paroi d'extrémité de l'embout 5, faisant saillie vers l'extérieur. L'écrou 17 possède une paroi intérieure 18 et une paroi extérieure 19 filetées. La paroi extérieure 19 coopère avec le filetage 16 du manche 15 afin de rendre solidaire ces deux éléments alors que la paroi intérieure 18 est visée sur un filetage formé à l'extrémité de la gaine 14 du tube flexible 4 afin de solidariser l'écrou 17 au tube 4. Dans un mode de réalisation, l'écrou 17 est réalisé en acier inoxydable.

De préférence, la cryode 3 est également équipée d'un manchon de protection 21 enveloppant et isolant une partie de l'embout 5, l'écrou 17 et l'extrémité du manche 15. Le manchon 21 est, par exemple, réalisé en silicone et permet notamment de protéger les parois latérales de l'embout 5 qui sont également refroidies et pourraient ainsi adhérer de manière non désirable à des tissues.

Dans un mode de réalisation préféré de l'invention, le dispositif 1 est équipé d'un tube rigide courbé 22 en polycarbonate permettant d'imposer une courbure à l'extrémité du tube flexible 4 afin d'améliorer l'ergonomie du dispositif 1. Ce mode de réalisation permet notamment de contourner le microscope utilisée lors de l'intervention chirurgicale.

Dans le mode de réalisation représenté sur la figure 3, le connecteur 2 est pourvu d'un orifice proximal 23 permettant l'introduction du gaz dans le conduit d'alimentation et d'un orifice latéral 24 pour l'évacuation du gaz. Le tube 4 est également solidarisé au connecteur 2 au moyen de colle cyanolite.

Afin de permettre une évacuation latérale du gaz, l'extrémité du conduit d'évacuation 12 est obturée par de la colle alors qu'un orifice latéral, non représenté, est formé dans le conduit d'évacuation 12 et disposé en coïncidence avec l'orifice latéral 24.

Par ailleurs, le connecteur 2 est muni de deux joints toriques 25, 26, disposés de part et d'autre de l'orifice latéral 24. En fonctionnement, l'orifice latéral 24 est connecté à un silencieux permettant la détente du gaz dans l'atmosphère.

L'orifice proximal 23 est quant à lui relié à un réservoir de gaz liquéfié (dioxyde d'azote ou de carbone) par l'intermédiaire d'une détendeur.

On notera que tous les matériaux utilisés doivent être biocompatible afin de permettre au dispositif d'être utilisé dans des conditions chirurgicales.

Par ailleurs, les dispositifs selon l'invention sont préalablement stérilisés. Avantageusement, la stérilisation est une stérilisation à l'oxyde d'éthylène à 45°. Cette stérilisation est réalisée selon des normes industrielles et nécessite des extracteurs qui ne sont pas habituellement disponibles dans les hôpitaux. Ce type de stérilisation est moins dommageable pour le dispositif 1 et permet l'utilisation de moyen de collage, tel que la colle cyanolite.

Après leur stérilisation, les dispositifs 1 sont ensuite conditionnés dans des emballages stériles et sont prêts à être acheminés vers le lieu de l'intervention chirurgicale.

L'invention est décrite dans ce qui précède à titre d'exemple. Il est entendu que l'homme du métier est à même de réaliser différentes variantes de réalisation de l'invention sans pour autant sortir du cadre de l'invention.

## Revendications

1. Dispositif cryogénique (1) à usage chirurgical permettant de congeler localement une zone du corps à une température inférieure à -50°C, le dispositif comprenant :
- un embout (5) comprenant une extrémité métallique (8) destinée à être mise en contact avec la zone à congeler et une chambre (7) de détente de gaz cryogénique formée par les parois de l'embout (5), lesquelles présentent une épaisseur homogène ;
- un tube flexible (4) dont une extrémité est insérée à l'intérieur de l'embout (5) et dans lequel on trouve :
- un conduit (12) d'évacuation du gaz cryogénique de la chambre ;
- un conduit (11) d'alimentation de la chambre en gaz cryogénique, ledit conduit d'alimentation (11) présentant une section inférieure au conduit d'évacuation en maintenant ainsi ledit gaz cryogénique sous pression avant son injection dans la chambre (7) de détente via une buse (6) d'injection;
- une buse (6) d'injection du gaz connectée au conduit (11) d'alimentation d'une part, et débouchant dans ladite chambre (7), en direction de l'extrémité métallique (8), d'autre part ;
ledit dispositif cryogénique (1) étant **caractérisé en ce que** le tube (4), le conduit (11) d'alimentation et le conduit (12) d'évacuation sont formés d'une seule pièce extrudée.

2. Dispositif cryogénique (1) selon la revendication 1, **caractérisé en ce que** ladite extrémité du tube (4) flexible est fixée à l'intérieur de l'embout et **en ce que** le dispositif cryogénique (1) comprend en outre :
- une source de gaz cryogénique communiquant avec le conduit d'alimentation (11), et apportant le gaz cryogénique à l'embout (5) pour congeler localement ladite zone du corps,
- un manche (15) en un matériau métallique isolant dont une extrémité distale est pourvue d'un filetage (16) coopérant avec un écrou (17) monté sur ledit embout (5).

3. Dispositif cryogénique (1) selon l'une des revendications précédentes, **caractérisé en ce que** l'embout (5) est solidarisé à une extrémité du tube (4) par des moyens de collage, préférentiellement une colle cyanolite.

4. Dispositif cryogénique (1) selon la revendication 3, **caractérisé en ce que** l'extrémité du tube flexible (4), insérée à l'intérieur de l'embout (5), est filetée afin de créer une rugosité à l'interface de collage entre l'embout (5) et le tube (4).

5. Dispositif cryogénique (1) selon l'une des revendications précédentes, **caractérisé en ce que** le conduit d'évacuation (12) présente sensiblement une forme de croissant.

6. Dispositif cryogénique (1) selon la revendication 5, **caractérisé en ce que** la concavité du croissant est orientée vers le conduit d'alimentation (11).

7. Dispositif cryogénique (1) selon l'une des revendications précédentes, **caractérisé en ce que** la buse d'injection (6) est insérée à l'intérieur du conduit d'alimentation (11).

8. Dispositif cryogénique (1) selon l'une des revendications précédentes, **caractérisé en ce que** la buse d'injection (6) possède une extrémité conique (21) d'injection.

9. Dispositif cryogénique (1) selon l'une des revendications précédentes, **caractérisé en ce qu'**il comprend un manchon (21) d'isolation recouvrant une portion de l'embout (5).

10. Dispositif cryogénique (1) selon l'une quelconque des revendications 2 à 9, **caractérisé en ce que** le tube (4) présente une gaine (14) extérieure de protection, ladite extrémité du tube (4) insérée et fixée à l'intérieur de l'embout (5) étant dénudée hors de ladite gaine (14).

11. Dispositif cryogénique (1) selon la revendication 10, **caractérisé en ce que** ledit écrou (17) possède une paroi intérieure (18) et une paroi extérieure (19) filetées, ladite paroi extérieure (19) coopérant avec ledit filetage (16) du manche (15) pour les rendre solidaires, et la paroi intérieure (18) est vissée sur un filetage formé à l'extrémité de la gaine (14) du tube flexible (4) pour rendre solidaires l'écrou (17) et le tube (4).

12. Dispositif (1) cryogénique selon l'une des revendications précédentes, **caractérisé en ce qu'**un manchon de protection (21) enveloppe et isole du froid une partie de l'embout (5), l'écrou (17) et ladite extrémité distale du manche (15).

13. Dispositif cryogénique (1) selon l'une des revendications précédentes, **caractérisé en ce que** le tube (4) a une longueur comprise entre 1,5 et 2,5 mètres, et de préférence de l'ordre de 2 mètres.

## Patentansprüche

1. Kryogene Vorrichtung (1) zur chirurgischen Verwendung, die ermöglicht, lokal einen Bereich des Köpers bei einer Temperatur von weniger als -50 °C tiefzukühlen, wobei die Vorrichtung Folgendes umfasst:
- ein Ansatzstück (5) mit einem metallischen Ende (8), das ausgelegt ist, um mit dem tiefzukühlenden Bereich in Kontakt zu kommen, und eine Kammer (7) zur Ausdehnung von kryogenem Gas, die von den Wänden des Ansatzstücks (5) gebildet ist, die eine gleichförmige Dicke aufweisen;
- ein flexibles Rohr (4), von dem ein Ende in des Innere des Ansatzstücks (5) eingeführt ist, und in dem Folgendes zu finden ist:
- eine Leitung (12) zur Evakuierung des kryogenen Gases aus der Kammer;
- eine Leitung (11) zur Versorgung der Kammer mit kryogenem Gas, wobei die Versorgungsleitung (11) einen Abschnitt aufweist, der unter der Evakuierungsleitung liegt, wobei auf diese Weise das kryogene Gas vor seiner Injizierung in die Ausdehungskammer (7) über eine Inkektionsdüse (6) unter Druck gehalten wird,
- eine Düse (6) zur Injizierung des Gases, die einerseits mit der Versorgungsleitung (11) verbunden ist und andererseits in Richtung des metallischen Endes (8) in die Kammer (7) mündet;
wobei die kryogene Vorrichtung (1) **dadurch gekennzeichnet ist, dass** das Rohr (4), die Versorgungsleitung (11) und die Evakuierungsleitung (12) aus einem einzigen extruiderten Stück gebildet sind.

2. Kryogene Vorrichtung (1) nach Anspruch 1, **dadurch gekennzeichnet, dass** das Ende des fliexiblen Rohrs (4) an das Innrere des Ansatzstücks befestigt ist, und dadurch, dass die kryogene Vorrichtung (1) außerdem Folgendes umfasst:
- eine Quelle von kryogenem Gas, die mit der Versorgungsleitung (11) in Verbindung steht und das kryogene Gas dem Ansatzstück (5) zuführt, um den Bereich des Körpers lokal tiefzukühlen,
- einen Griff (15) aus einem isolierenden metallischen Material, von dem ein distales Ende mit einem Gewinde (16) versehen ist, das mit einer Schraubenmutter (17) zusammenarbeitet, die auf dem Ansatzstück (5) montiert ist.

3. Kryogene Vorrichtung (1) nach einem der vorhergehenden Ansprüche, **dadurch gekennzeichnet, dass** das Ansatzstück (5) mit einem Ende des Rohrs (4) mit Klebemitteln, vorzugsweise einem Cyanolit-Kleber, fest verbunden ist.

4. Kryogene Vorrichtung (1) nach Anspruch 3, **dadurch gekennzeichnet, dass** das Ende des flexiblen Rohrs (4), das in das Innere des Ansatzstücks (5) eingeführt ist, gewindegeschnitten ist, um eine Rauhigkeit an der Klebeschnittstelle zwischen dem Ansatzstück (5) und dem Rohr (4) zu bilden.

5. Kryogene Vorrichtung (1) nach einem der vorhergehenden Ansprüche, **dadurch gekennzeichnet, dass** die Evakuierungsleitung (12) im Wesenlichen halbmondförmig ist.

6. Kryogene Vorrichtung (1) nach Anspruch 5, **dadurch gekennzeichnet, dass** die Konkavität des Halbmondes auf die Versorgungsleitung (11) hin ausgerichtet ist.

7. Kryogene Vorrichtung (1) nach einem der vorhergehenden Ansprüche, **dadurch gekennzeichnet, dass** die Injektionsdüse (6) in das Innere der Versorgungsleitung (11) eingeführt ist.

8. Kryogene Vorrichtung (1) nach einem der vorhergehenden Ansprüche, **dadurch gekennzeichnet, dass** die Injektionsdüse (6) ein konisches Injektionsende (21) aufweist.

9. Kryogene Vorrichtung (1) nach einem der vorhergehenden Ansprüche, **dadurch gekennzeichnet, dass** sie eine Isoliermuffe (21) umfasst, die einen Teil des Ansatzstücks (5) abdeckt.

10. Kryogene Vorrichtung (1) nach einem der Ansprüche 2 bis 9, **dadurch gekennzeichnet, dass** das Rohr (4) eine äußere Schutzhülle (14) aufweist, wobei das Ende des Rohrs (4), das in das Innere des Ansatzstücks (5) eingeführt und daran befestigt ist, außerhalb der Hülle (14) feigelegt ist.

11. Kryogene Vorrichtung (1) nach Anspruch 10, **dadurch gekennzeichnet, dass** die Schraubenmutter (17) eine gewindegeschnittene innere (18) und äußere (19) Wand umfasst, wobei die äußere Wand (19) mit dem Gewinde (16) des Griffs (15) zusammenarbeitet, um sie fest miteinander zu verbinden, und die innere Wand (18) auf ein Gewinde geschraubt ist, das am Ende der Hülle (14) des flexiblen Rohrs (4) gebildet ist, um die Schraubenmutter (17) und das Rohr (4) fest miteinander zu verbinden.

12. Kryogene Vorrichtung (1) nach einem der vorhergehenden Ansprüche, **dadurch gekennzeichnet, dass** eine Schutzmuffe (21) einen Teil des Ansatzstücks (5), die Schraubenmutter (17) und das distale Ende des Griffs (15) umhüllt und von der Kälte isoliert.

13. Kryogene Vorrichtung (1) nach einem der vorhergehenden Ansprüche, **dadurch gekennzeichnet, dass** das Rohr (4) eine Länge aufweist, die zwischen 1,5 und 2,5 Metern und vorzugsweise im Bereich von 2 Metern liegt.

## Claims

1. Cryogenic device (1) for surgical use suitable for locally freezing an area of the body at a temperature below -50°C, the device comprising:
- a tip (5) comprising a metallic end (8) intended to be placed in contact with the area to be frozen and a cryogenic gas expansion chamber (7) formed by the walls of the tip (5) which have a homogeneous thickness;
- a flexible tube (4) wherein one end is inserted inside the tip (5) and containing:
- a cryogenic gas discharge conduit (12) from the chamber;
- a conduit (11) for supplying the chamber with cryogenic gas, said supply conduit (11) having a cross-section less than the discharge conduit by thus keeping said cryogenic gas compressed before the injection thereof into the expansion chamber (7) via an injection nozzle (6);
- a nozzle (6) for injecting gas connected to the supply conduit (11), on one hand, and opening into said chamber (7), towards the metallic end (8), on the other;
said cryogenic device (1) being **characterised in that** the tube (4), the supply conduit (11) and the discharge conduit (12) are formed from a single extruded part.

2. Cryogenic device (1) according to claim 1, **characterised in that** said end of the flexible tube (4) is attached inside the tip and **in that** the cryogenic device (1) further comprises:
- a cryogenic gas source communicating with the supply conduit (11), and supplying the cryogenic gas to the tip (5) to locally freeze said area of the body,
- a sleeve (15) made of an insulating metallic material wherein one distal end is provided with a thread (16) engaging with a nut (17) mounted on said tip (5).

3. Cryogenic device (1) according to any of the above claims, **characterised in that** the tip (5) is rigidly connected to one end of the tube (4) by bonding means, preferentially a cyanolite adhesive.

4. Cryogenic device (1) according to claim 3, **characterised in that** the end of the flexible tube (4), inserted inside the tip (5) is threaded so as to create roughness at the bonding interface between the tip (5) and the tube (4).

5. Cryogenic device (1) according to any of the above claims, **characterised in that** the discharge conduit (12) has substantially a crescent shape.

6. Cryogenic device (1) according to claim 5, **characterised in that** the concavity of the crescent is oriented towards the supply conduit (11).

7. Cryogenic device (1) according to any of the above claims, **characterised in that** the injection nozzle (6) in inserted inside the supply conduit (11).

8. Cryogenic device (1) according to any of the above claims, **characterised in that** the injection nozzle (6) has a conical injection end (21).

9. Cryogenic device (1) according to any of the above claims, **characterised in that** it comprises an insulating sleeve (21) covering a portion of the tip (5).

10. Cryogenic device (1) according to any of claim 2 to 9, **characterised in that** the tube (4) has an outer protective sheath (14), said end of the tube (4) inserted and fixed inside the tip (5) being exposed outside said sheath (14).

11. Cryogenic device (1) according to claim 10, **characterised in that** said nut (17) has an inner wall (18) and an outer wall (19) which are threaded, said outer wall (19) engaging with said thread (16) of the sleeve (15) to render same rigidly connected, and the inner wall (18) is screwed onto a thread formed at the end of the sheath (14) of the flexible tube (4) to render the nut (17) and the tube (4) rigidly connected.

12. Cryogenic device (1) according to any of the above claims, **characterised in that** a protective sleeve (21) encases and insulates from the cold a portion of the tip (5), the nut (17) and said distal end of the sleeve (15).

13. Cryogenic device (1) according to any of the above claims, **characterised in that** the tube (4) has a length between 1.5 and 2.5 metres, and preferably in the region of 2 metres.
